# EUROPEAN PATENT SPECIFICATION

(11) **EP 1 383 861 B1**
(45) Date of publication and mention of the grant of the patent: **03.05.2006**
(21) Application number: 02706130.8
(22) Date of filing: 05.02.2002
(51) Int. Cl.: C12M 1/12, C12N 1/02

(54) **FILTRATION ASSEMBLY**
FILTRATIONSGERÄT
ENSEMBLE DE FILTRATION

(30) Priority: 06.02.2001 US 266487 P
(43) Date of publication of application: 28.01.2004
(73) Proprietor: PALL CORPORATION, East Hills, NY 11548-1209 (US)
(72) Inventor: KANE, Jeffrey, Manchester, MI 48158 (US)
(74) Representative: Hoeger, Stellrecht & Partner Patentanwälte
(86) International application number: PCT/US2002/003165
(87) International publication number: WO 2002/062942

(56) References cited:
- EP-A- 0 478 914
- WO-A-01/48142
- WO-A-96/37600
- WO-A-98/32875
- US-A- 3 448 011
- US-A- 4 340 480
- US-A- 4 777 137
- US-A- 5 578 459

## Description

### FIELD OF THE INVENTION

This invention relates to a filtration assembly for collecting a fluid sample possibly containing microorganisms, and capturing the microorganisms of interest in the sample such that the captured microorganisms can be subsequently detected.

### BACKGROUND OF THE INVENTION

A common method for determining the presence of microorganisms in a fluid includes collecting a fluid sample in a first container and subsequently transferring it to a filter device including a filter element. The sample is then passed through the filter element which is capable of capturing the microorganisms larger than a certain size. After filtration of the sample, the filter element with the captured microorganisms is transferred to a petri dish containing a nutrient solution that supports the growth of the microorganisms. The nutrient solution permeates through the filter element to reach the microorganisms, enabling the microorganisms to be cultured atop the filter element.

This method is labor intensive, and can introduce external contaminants (e.g., microorganisms) into the sample that were not originally present. Accordingly, a fluid sample can be incorrectly determined to be contaminated with microorganisms.

The present invention provides for ameliorating at least some of the disadvantages of the prior art. These and other advantages of the present invention will be apparent from the description as set forth below.

### BRIEF SUMMARY OF THE INVENTION

In accordance with an embodiment of the invention, a filtration assembly is provided comprising a sample reservoir for receiving a fluid sample to be filtered, a removable cover, and at least one vent including a liquophobic element communicating with the chamber, wherein the assembly further comprises a fluid port communicating with the chamber, and a filter element disposed in a flow path between the sample reservoir and the fluid port.
US 3,448,011 discloses a bacteriological filtration and incubation apparatus to test fluids for microorganisms. The apparatus comprises a container having a filter support bed upon which a filter membrane may be seated during a filtration process. The filter membrane is adapted to collect a microorganism from a fluid to be tested which is introduced into the container. The container is covered by a lid including an opening to allow gaseous communication with the atmosphere through a bacteriological filter.
WO 98/32875 describes a filter assembly for use in culturing microorganisms including a sample reservoir for holding a fluid to be filtered. At the lower end of the reservoir, a filter element is supported whereas the upper end is closed by a cover assembly.

In another embodiment of the present invention, a filtration assembly is provided comprising a sample reservoir for holding a fluid sample to be filtered, a base detachably mounted to the sample reservoir including a fluid port and a filter support surface for supporting a filter element, and a removable cover member including a vent comprising a liquophobic element, wherein the fluid port and vent communicate with the sample reservoir.

In some embodiments of the filtration assembly, the filter element is removably disposed in a fluid flow path between the sample reservoir and the fluid port.

In accordance with another embodiment of the present invention, a method of filtering a fluid is provided comprising collecting a fluid to be filtered in a sample reservoir, connecting a detachable cover member to the sample reservoir, drawing the fluid to be filtered through a filter medium disposed adjacent the sample reservoir while drawing air into the sample reservoir through a vent and removing the fluid which has passed through the filter element from a fluid port communicating with the sample reservoir.

In another embodiment, a method of culturing microorganisms is provided comprising collecting a microorganism-containing fluid to be filtered in a sample reservoir, connecting a detachable cover member to the sample reservoir, drawing the fluid to be filtered through a microorganism-capturing filter medium disposed adjacent the sample reservoir while drawing air into the sample reservoir through a vent, removing the fluid which has passed through the filter element from a fluid port communicating with the sample reservoir, and incubating the microorganisms captured by the filter medium.

### BRIEF DESCRIPTION OF THE DRAWINGS

Figure 1 is an isometric view of an embodiment of the filtration assembly of the present invention.

Figure 2 is vertical cross-sectional view of an embodiment of the sample reservoir of the present invention.

Figure 3 is a vertical cross-sectional view of an embodiment of the cover of the present invention.

Figure 4 is a vertical cross-sectional view of an embodiment of the filtration assembly of the present invention of the base of the present invention.

Figure 5 is a vertical cross-sectional view of an embodiment of the base of the present invention.

Figure 6 is a top isometric view of an embodiment of the base of the present invention.

Figure 7 is a bottom isometric view of an embodiment of the base of the present invention.

Figure 8 illustrates a vacuum filtering arrangement with which embodiments of the present invention can be used.

Figure 9 is a vertical cross-sectional view of an embodiment of the base installed on a vacuum manifold using an adapter and a stopper.

Figure 10 is a vertical cross-sectional view of an embodiment of the base directly engaging a vacuum manifold for vacuum filtration.

Figure 11 is a bottom isometric view of the base illustrating a method of introducing a nutrient solution through the fluid port of the base.

### DETAILED DESCRIPTION OF THE INVENTION

In accordance with an embodiment of the invention, a filtration assembly is provided comprising a sample reservoir for receiving a fluid to be filtered, a removable cover and a vent including a liquophobic element communicating with the sample reservoir, the assembly further comprising a fluid port communicating with the sample reservoir, the assembly defining a fluid flow path between the sample reservoir and the fluid port, and a filter element for capturing microorganisms of interest in the fluid sample, the filter element being disposed across the fluid flow path between the sample reservoir and the fluid port. In accordance with this embodiment, the cover is removed to allow the sample to be collected in the sample reservoir, and the cover is replaced after the sample is collected. Subsequently, the fluid is drawn from the covered reservoir through the filter element and fluid port, while the vent allows air, but not contaminants (e.g., microorganisms, such as bacteria) from the environment external to the sample reservoir to be passed into the reservoir.

A filtration assembly according to an embodiment of the invention comprises a sample reservoir for holding a fluid sample to be filtered, a cover member detachably covering the sample reservoir with a fluid-tight fit, at least one vent in communication with the sample reservoir, wherein the vent includes a liquophobic element that allows air into the sample reservoir but prevents the passage of microorganisms into the sample reservoir, a fluid port in fluid communication with the sample reservoir, and a filter element disposed in a flow path between the sample reservoir and the fluid port. The cover member and/or the sample reservoir (e.g., a wall of the reservoir) can include the vent. In one preferred embodiment, the filtration assembly includes a base detachably mounted to the sample reservoir, the base including the fluid port, and a filter support surface for supporting a filter element.

In another embodiment, filtration assembly is provided comprising a sample reservoir for holding a fluid sample to be filtered, a base detachably mounted to the sample reservoir including a fluid port in communication with the sample reservoir, the base including a filter support surface for supporting a filter element, a cover member detachably covering the sample reservoir with a fluid-tight fit, wherein the cover member includes a vent communicating with the sample reservoir, the vent comprising a liquophobic element that allows air into the sample reservoir and prevents the passage of microorganisms into the sample reservoir.

A method of filtering a fluid according to an embodiment of the invention comprises collecting a fluid to be filtered in a sample reservoir, connecting a detachable cover member to the sample reservoir, drawing the fluid to be filtered through a filter element disposed adjacent the sample reservoir and drawing air into the reservoir through a vent, and removing the fluid which has passed through the filter element from a fluid port.

A method of culturing microorganisms according to an embodiment of the invention comprises collecting a microorganism-containing fluid to be filtered in a sample reservoir, connecting a detachable cover member to the sample reservoir, drawing the fluid to be filtered through a microorganism-capturing filter medium disposed adjacent the sample reservoir while drawing air into the sample reservoir through a vent, removing the fluid which has passed through the filter element from a fluid port communicating with the sample reservoir, and incubating the microorganisms captured by the filter medium.

A method of using a filtration assembly according to an embodiment of the invention comprises placing a base of a filtration assembly on a vacuum manifold with a skirt of the base contacting an inlet tube of the manifold around a periphery of the skirt, wherein the filtration assembly further comprises a vent, applying suction to the interior of the inlet tube to draw a fluid to be filtered through a filter element within the filtration assembly and into the manifold and to draw air through the vent into the filtration assembly.

Methods for filtering fluid, culturing microorganisms, and using a filtration assembly according to the invention can include passing air into the reservoir through a vent disposed in the cover member and/or passing air into the reservoir through a vent in or on the reservoir.

Embodiments of the invention are suitable for use with a variety of fluids. For example, fluids in the biopharmaceutical, microelectronics, and beverage industries may be filtered in accordance with embodiments of the invention.

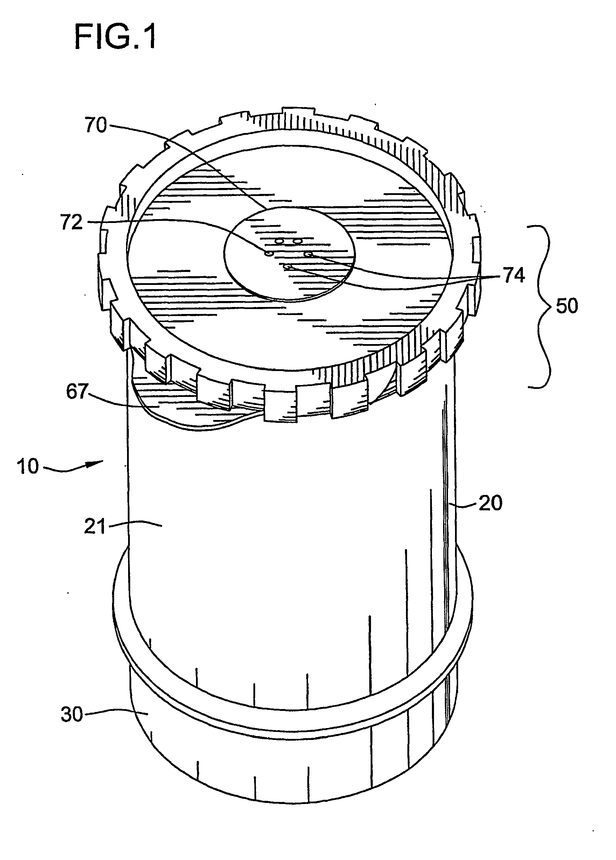

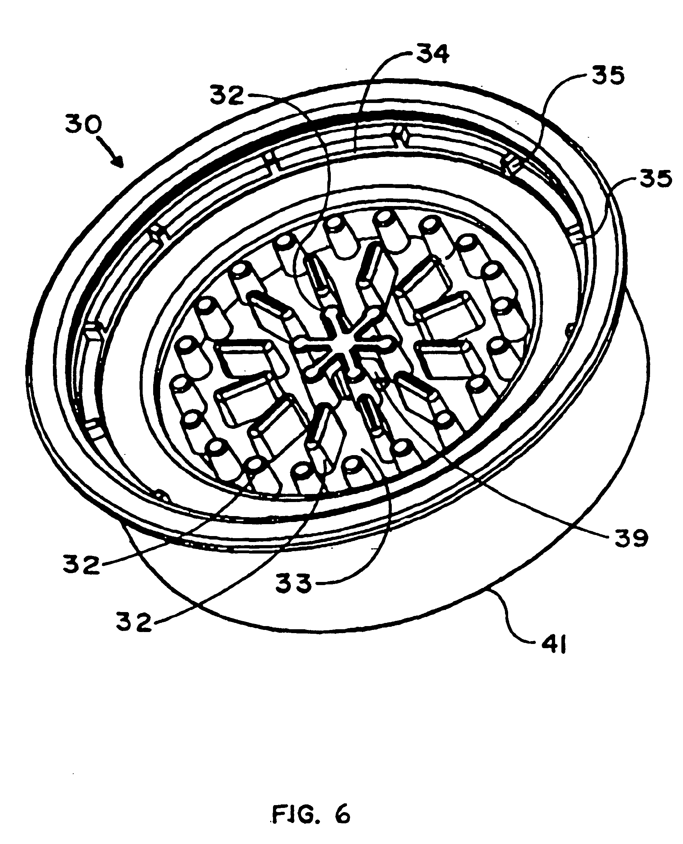

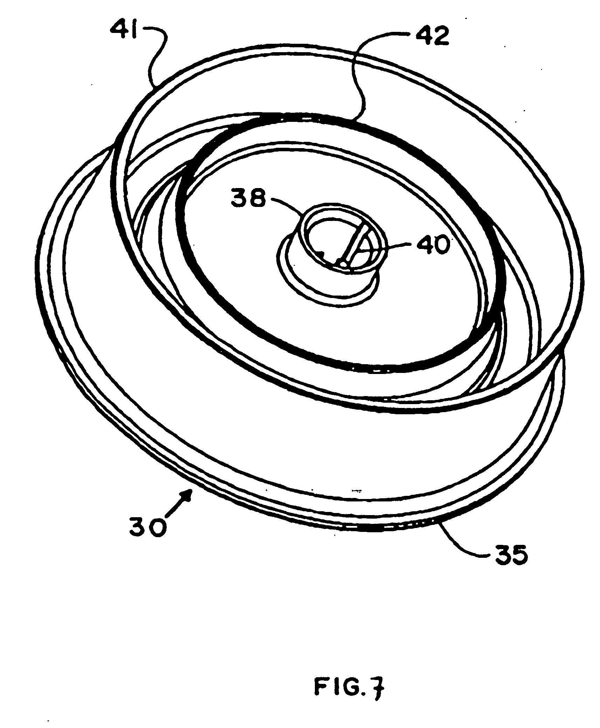

An embodiment of a filtration assembly 10 according to the present invention is illustrated in Figures 1-7. As shown in these figures, the assembly 10 preferably includes a sample reservoir 20 and a base 30 which may be detachably engageable with the lower end of the sample reservoir 20. The sample reservoir 20 defines a chamber 22 including an outer wall 21 and an outer wall 27, and the chamber 22 may be used to collect and hold a fluid sample which is to be filtered. In the illustrated embodiment, the base 30 serves to support the sample reservoir 20 and includes a fluid port 38. A filter 45 is preferably disposed in a fluid flow path between the sample reservoir 20 and the fluid port 38 so that the fluid sample to be filtered passes from the reservoir and through the filter and the fluid port. A cover member 50 is preferably detachably engageable with the upper end of the sample reservoir 20 and forms a fluid-tight seal between the cover member 50 and the sample reservoir 20. In this illustrated embodiment, the cover member 50 includes a vent 70 comprising a liquophobic element 72 which allows the passage of gas (e.g., air) but prevents the passage of liquid or microorganisms there through. However, the vent 70 need not be associated with the cover member 50, it may, for example, be associated with a wall of the sample reservoir 20 (e.g., the outer wall 21, or the inner wall 27).

The sample reservoir 20 may have any structure which enables it to hold a desired volume of a sample fluid which is to be filtered. In the illustrated embodiment, the sample reservoir 20 is generally cylindrical and is open at its upper and lower ends. The sample reservoir 20 has an outer wall 21 which defines the outer periphery of the sample reservoir 20 for the sample fluid, and an inner wall 27 that defines the inner periphery of the reservoir 20. The outer wall 21 may have a circular transverse cross-sectional shape and an inner diameter (provided by inner wall 27) which linearly decreases from its upper to its lower end. The shapes of the outer wall 23 and inner wall 27 are not critical and the diameter need not vary over its height. For example, the transverse cross-sectional shape may be polygonal or of a non-circular curved shape and the inner diameter or other dimensions of the sample reservoir 20 may be constant or vary in any desired manner over the height of the sample reservoir 20. The sample reservoir 20 may include gradations on its inner or outer surface to assist a user in measuring the amount of sample fluid being collected.

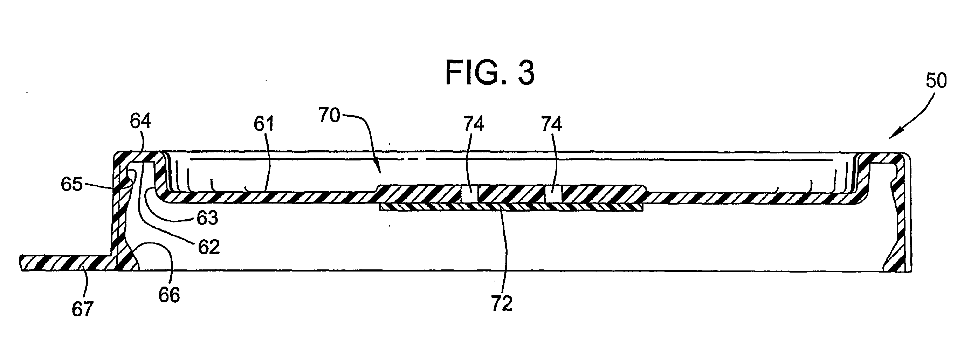

The filtration assembly 10 includes a cover member 50. A preferred embodiment of the cover 50, which is best illustrated in Figure 3, is shaped so as to detachably fit atop the upper end of the sample reservoir 20. The cover 50 may engage with the upper end of the sample reservoir 20 in various manners. For example, they may engage each other with a snap fit, a bayonet fit, threaded engagement, press fit, or a loose fit. Preferably, however, the engagement provides a fluid-tight seal between the cover and the reservoir, and the engagement is such as to provide some resistance to disengagement of the cover 50 from the sample reservoir 20 so as to enable the filtration assembly 10 to be handled and transported without the cover 50 falling off the sample reservoir 20, while still permitting the cover 50 to be readily detached from the sample reservoir 20. Additionally, the cover member 50 may be shaped so as to be detachably connectable to the base 30, for example, when the cover member 50 and base 30 together are to be used as part of a petri dish.

The filtration assembly 10 includes at least one vent 70 comprising at least one, and preferably a plurality of, ports 74, and a liquophobic element 72. Including a vent allows the fluid to be filtered to be withdrawn from the filtration assembly 10 without removing the cover 50 and exposing the sample to contaminants. In cases in which the fluid is withdrawn using suction, the inclusion of a vent 70 prevents the sample reservoir 20 from collapsing under the suction.

In the illustrated embodiment, the cover 50 includes the vent 70, and the vent includes five ports 74. Liquophobic element 72 disposed in the flow path through the vent 70, which prevents the passage of external environmental contaminants, e.g., microorganisms, liquids, dust, etc., into the sample reservoir 20, but allows the passage of air through the port(s) 74 into the sample reservoir 20 after the sample is collected and the cover 50 is fluid tightly engaged with the reservoir 20. Element 72 can be attached to the filtration assembly, e.g., to cover 50 and/or a wall of the sample reservoir 20, as is known in the art.

Typically, the liquophobic element 72 has a pore structure, e.g., a pore size (for example, as evidenced by bubble point, or by K_{L} as described in, for example, U.S. Patent No. 4,340,479), a pore diameter, a pore rating, or a fine particle arresting efficiency (as described in, for example, the Monodisperse Dioctyl Phthalate (DOP) Smoke Test (ASTM D2986-71, or ASTM D 2986-95a)) that reduces the passage therethrough of microorganisms, preferably reducing the passage of bacteria therethrough. For example, in some embodiments, the liquophobic element 72 has a pore size of about 2.0 micrometers or less, or about 1.0 micrometer or less. Alternatively, in some embodiments, the liquophobic element 72 removes at least about 99.9% of particles having diameters of at least about 0.2 microns (µm) or greater, or the element removes at least about 99.97% of particles having diameters of at least about 0.3 µm or greater. Illustratively, in one embodiment, the liquophobic element has a Monodisperse Dioctyl Phthalate (DOP) Smoke Penetration (e.g., as measured by ASTM D2986-71) at 0.3 µm at 10.5 ft/minute gas flow of at least about 0.03%.

The liquophobic element can be of any desired type, such as a microporous membrane (e.g., commercially available from Pall Life Sciences (Ann Arbor, MI) and Pall Corporation (East Hills, NY)), or a porous plastic member, e.g., POREX® porous plastic components commercially available from Porex Corporation (Fairburn, GA). Preferably, the liquophobic element comprises a hydrophobic microporous membrane. Suitable liquophobic elements including elements having a bacterial blocking pore structure are commercially available.

Although in the illustrated embodiment, the vent 70 is located on the surface of cover 50 facing the interior of the sample reservoir 20, the vent 70 may be disposed in other locations. For example, the vent 70 can be disposed in or on the outer wall 21 or the inner wall 27 of the sample reservoir 20, or the vent can be in or on other portions of the cover. Additionally, the vent can include any suitable number of ports, and the ports can have any suitable inner diameters.

Prior to use, the vent 70 may be covered with a removable element, for example, to prevent exposing the element 72 to undesirable material (e.g., contaminants, sterilizing agents, etc.) before use. The vent 70 may be covered using any suitable material including, for example, a sticker, label or seal and/or an additional cover member. A sticker covering the vent 70 preferably utilizes an adhesive providing some resistance to removal but still allowing easy removal. The sticker may be sized to cover only the vent 70 or alternatively may extend beyond the area of the vent. The sticker may contain pre-printed indicia and/or may comprise a material upon which a user may mark or write. Additionally or alternatively, an additional cover member may engage with the cover 50 in various manners and with varying degrees of tightness as described above with respect to the sample reservoir 20 and the base 30 and the cover 50. The additional cover member may also be shaped so as to fit atop the upper end of the base 30, thereby enabling the additional cover and the base 30 together to form a petri dish.

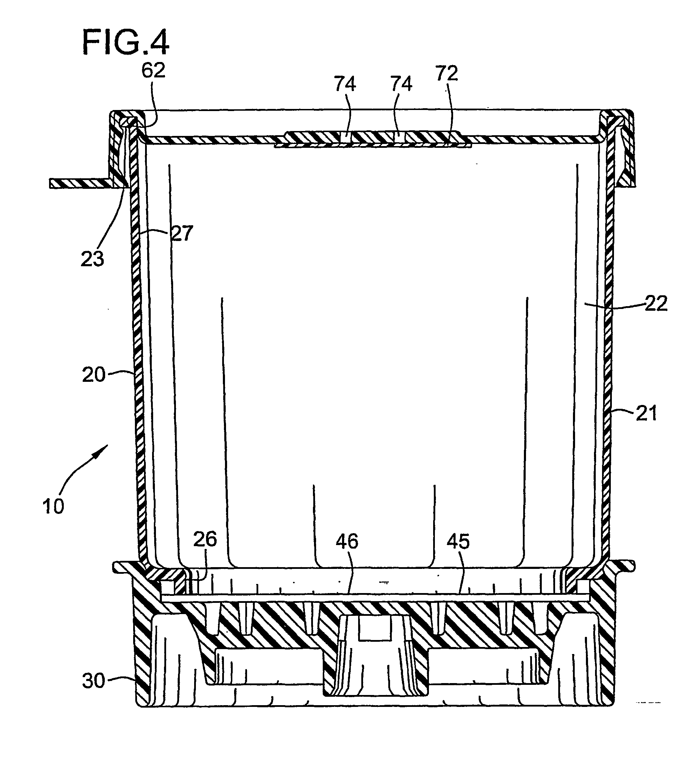

In the illustrated embodiment, the cover 50 that includes vent 70 comprises a disc-shaped plate 61 including a tab 67 protruding from the outer periphery of the plate 61. The cover 50 includes a continuous annular inverted channel 62 formed around the entire outer periphery of the disc-shaped plate 61. As shown in more detail in Figure 3, the inverted channel 62 preferably comprises an inner wall 63 extending upwards from the upper surface of the disc-shaped plate 61, a generally right angular ledge 64 around the top edge of inner wall 63 and extending outwardly from the center of the disc-shaped plate 61 and an outer wall 65 extending downwardly from the right annular ledge 64. Using the embodiment shown in Figure 4 for reference, a snap-fit is formed between the inverted channel 62 and a radially outward lip 23 formed around the entire outer periphery of the upper end of the sample reservoir 20. The outer wall 65 has a radially inward bulge 66. The outer diameter of the inverted channel 62 measured at the bulge 66 in a relaxed (unstressed) state is smaller than the outer diameter of the sample reservoir 20 at the lip 23 in a relaxed state so that once the lip 23 is urged upwards past the bulge 66, the bulge 66 will resist disengagement of the sample reservoir 20 and the cover 50. The engagement between the cover 50 and the sample reservoir 20 may be of varying degrees of tightness. For example, the engagement may be sufficient to provide some resistance to disengagement without forming a seal, or preferably, the engagement provides a fluid-tight seal between the two members.

A fluid-tight seal between the cover 50 and the sample reservoir 20 is convenient when the sample reservoir 20 is to be used for collecting and/or temporary storage of a fluid sample prior to filtration. For example, in factories and fluid processing facilities, it is common to collect a fluid sample in one part of the factory or facility and then to carry the sample to a laboratory for analysis in a different part of the factory or facility. In such cases, the provision of a fluid-tight seal between the cover 50 and the sample reservoir 20 (and the configuration of the vent) enables a fluid sample within the sample reservoir 20 to be transported from one location to another without fear of spilling or contamination. The provision of tab 67 facilitates disengagement of the cover 50 from the reservoir 20, for example, to introduce the sample into the sample reservoir. A fluid-tight seal can be formed by any suitable means, but preferably by one which does not require the use of a separate sealing member, such as an O-ring or a gasket. In the illustrated embodiment, a fluid-tight seal is achieved between the cover 50 and the sample reservoir 20 by the inverted channel 62. The inner diameter of the inverted channel 62 in a relaxed state is larger than the inner diameter of the upper end of the sample reservoir 20 in a relaxed state so that when the lip 23 of the sample reservoir 20 is placed into the inverted channel 62, the upper end of the sample reservoir 20 will be urged radially outwards by the inner wall 63 of the inverted channel 62 towards the outer wall 65 of the inverted channel 62. The upper end of the sample reservoir 20 is thereby pressed into intimate contact with the inverted channel 62, resulting in the formation of a fluid-tight seal between the cover 50 and the sample reservoir 20 around the entire periphery of the sample reservoir 20.

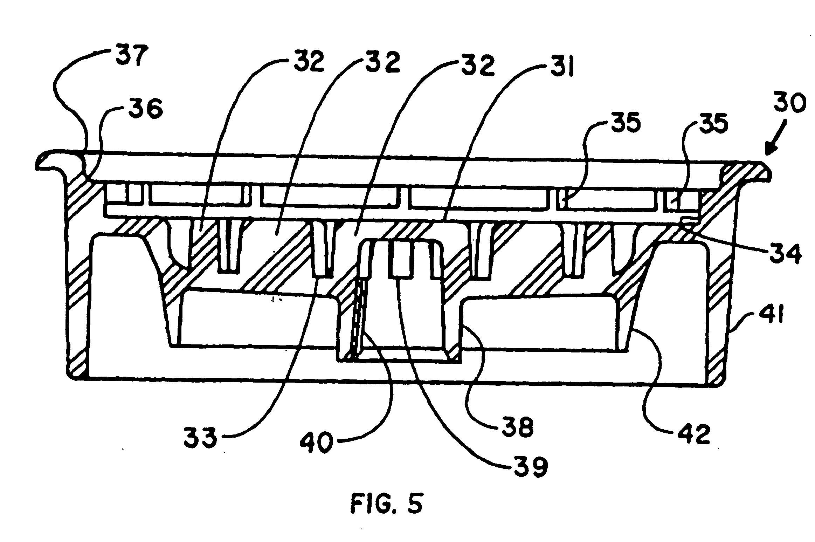

In the illustrated embodiment, the filtration assembly includes a base 30. The base 30 preferably provides support for the sample reservoir 20. The base 30 may also include a fluid port and/or a filter support surface 31. As illustrated in Figures 4 and 5, which are respectively a vertical cross-sectional view and a top isometric view of the base 30, the base 30 includes a filter support surface 31 and a fluid port 38. The filter support surface 31 is defined by the upper surfaces of a plurality of projections 32 which extend upwards from a bottom inner surface 33 of the base 30. The projections 32 are spaced from each other to enable filtrate which has passed through the filter element 45 to flow between the projections 32 and out through the fluid port 38. One or more drainage openings 39 for filtrate are formed in the projections 32 at the center of the base 30 to connect the interior of the fluid port 38 with the region of the base containing the projections 32.

In the embodiment shown in the Figures, the base 30 is a unitary member formed by injection molding, for example, with the filter support surface 31 being integrally formed with other portions of the base 30. However, it is also possible for the base 30 to comprise a plurality of separately formed components. For example, the filter support surface 31 may comprise a perforated plate, a porous plate, or a mesh which is removably installed within the interior of the base 30 and has an upper surface which can support the filter element 45.

The filter support surface 31 as illustrated is planar, but it may have any shape which enables it to support the filter element 45 for filtration. For example, the filter support surface 31 may be dished, arched or wave-like in shape.

In the illustrated embodiment, the filter support surface 31 is surrounded by a circular wall 34 extending upwards from the outer periphery of the filter support surface 31, and a plurality of radial projections 35 extend upwards from a ledge formed atop the wall 34, with the vertical radially inner surface of each projection 35 being flush with the wall 34. The wall 34 and the projections 35 serve to surround and position a filter element 45 disposed on the filter support surface 31.

Although the filter support surface 31 is shown as a component of the base 30, embodiments of the support surface are not so limited. The filter support surface 31 may be formed or positioned within the sample reservoir 20. For example, instead of the sample reservoir 20 being completely open at its lower end, it may have a perforated bottom surface for supporting a filter element 45. Alternatively, the filter support surface 31 may be omitted entirely, for example, if the filter is self-supporting.

Preferably, the filtration assembly 10 is capable of standing upright on a level surface without being supported. In the illustrated embodiment, the base 30 includes an outer wall 41 extending around its entire periphery for supporting the base 30 on a table or other level surface. However, members other than a continuous wall can also be used to support the base, such as a plurality of legs. Alternatively, the base 30 may not be self-supporting, and it may have a shape which does not stand upright by itself. For example, the bottom of the base 30 may be shaped like a funnel.

The sample reservoir 20 and the base 30 may have a variety of configurations. In one embodiment, the sample reservoir 20 and the base 30 may be separately formed and permanently connected to each other, or they may be formed as a single member. In another embodiment, the filtration assembly 10 may not include a base 30. However, in the illustrated embodiment, the sample reservoir 20 is detachably engaged with the base 30 so that the base 30 can be separated from the sample reservoir 20, for example, to remove the filter element 45 or so that the base 30 can be used separately from the sample reservoir 20 as part of a petri dish.

The manner of engagement between the sample reservoir 20 and the base 30 is preferably such that the engagement creates a fluid-tight seal without the need for a sealing member, such as an O-ring or gasket, yet such that the sample reservoir 20 and the base 30 can be readily disengaged from each other by hand. The lower end of the sample reservoir 20 is also preferably shaped so that a fluid-tight seal is formed between the sample reservoir 20 and the upper surface of a filter element 45 disposed on the filter support surface 31 to prevent fluid from the sample reservoir 20 from bypassing the filter element 45 by flowing between the sample reservoir 20 and the filter element 45.

Advantageously, any type of detachable engagement providing intimate, sealing contact between the sample reservoir 20 and the base 30 around the entire inner periphery of the base 30 may be employed to detachably engage the two members. For example, there may be an interference fit between the sample reservoir 20 and the base 30 so that a radial force presses a peripheral surface of the sample reservoir 20 into sealing contact with an opposing peripheral surface of the base 30, or opposing surfaces of the sample reservoir 20 and the base 30 may be pressed into sealing contact with each other by a compressive force acting in the axial direction of the filtration assembly 10. In the illustrated embodiment, the sample reservoir 20 and the base 30 are engaged with each other by an interference fit which produces a fluid-tight seal between the outer peripheral surface of the sample reservoir 20 and the inner peripheral surface of the base 30. The sample reservoir 20 and the base 30 may be structured so as to provide resistance to an axial force tending to pull them apart so as not to be inadvertently disconnected from each other during use.

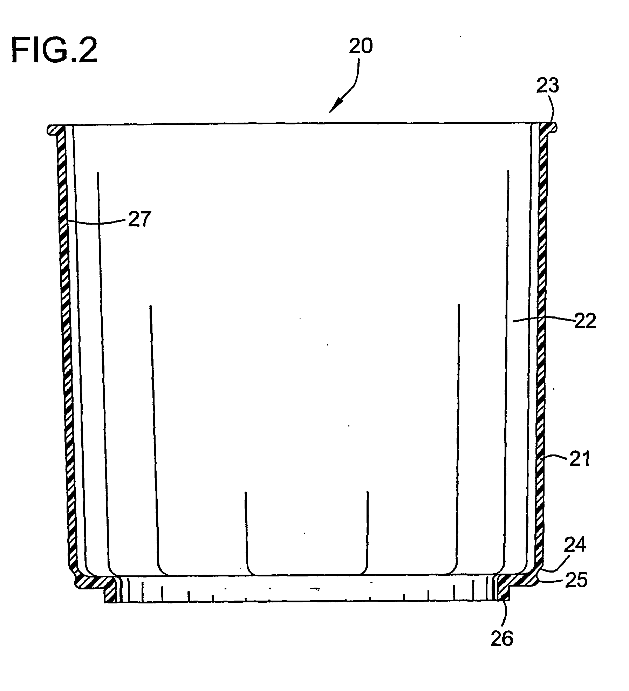

In the present embodiment, resistance to disengagement is provided by a snap fit in which the lower end of the sample reservoir 20 is received inside the upper end of the base 30. As shown in the cross-sectional elevation of Figure 2, the lower end of the sample reservoir 20 has a groove 24 and a radially outward projection 25 which extend continuously around its entire outer periphery. Similarly, as shown in Figure 5, the base 30 has a groove 36 and a radial inward projection 37 extending continuously around its entire inner periphery at its upper end. The outer diameter of the lower end of the sample reservoir 20 and the inner diameter of the base 30 are preferably selected so that the projections 25 and 37 will snap into and fit snugly inside the grooves 36 and 24, respectively, with an interference fit so that there is intimate contact, such as line contact or surface contact, between each projection and the corresponding groove around the entire circumference of the sample reservoir 20. The sample reservoir 20 may be disconnected from the base 30 simply by flexing the two members with respect to each other, for example, to disengage the projections from the grooves.

It is generally easier to disengage the two members if the groove 36 and the projection 37 are formed as close to the upper end of the base 30 as possible. For example, in the illustrated embodiment, projection 37 immediately adjoins the upper end of the base 30. The location of the sealing contact between the sample reservoir 20 and the base 30 is not critical so long as the contact can prevent fluid from leaking to the exterior of the filtration assembly 10 during normal use. For example, the sealing contact may be between the mating surfaces of the grooves 24, 36 and the projections, 25, 37 or it could be formed in a different location, with engagement between the grooves and the projections serving primarily to resist inadvertent disengagement of the sample reservoir 20 and the base 30 or to maintain an axial compressive force between the sample reservoir 20 and the filter element 45 to form a fluid-tight seal against the filter element 45. In the latter case, the grooves and the projections need not be continuous members.

In the illustrated embodiment, each groove 24,36 is complementary in shape with the corresponding projection 25,37, i.e., it has substantially the same radius of curvature as the corresponding projection so that each groove and the corresponding projection are in surface contact, but the curvatures of the groove and the projection may alternatively be such that they are in line contact, for example. It is possible to form a seal between the sample reservoir 20 and the base 30 with a single projection formed on the surface of one of the two members and a single groove for engagement with the projection formed on the surface of the other two members, but a plurality of grooves and projections may create a seal of greater integrity.

Many other arrangements besides a snap fit can be used to resist disengagement between the sample reservoir 20 and the base 30, such as a bayonet fit or threaded engagement. It may also be desirable to dispose tape or a sleeve, such as a shrink wrap sleeve, around the joint between the sample reservoir 20 and the base 30 or to lightly weld or bond the two members to each other (such as by ultrasonic welding) around their peripheries to secure the members together while enabling them to be easily disconnected from each other when desired. Such a manner of connection may be employed instead of or in addition to the interference fit provided by the grooves 24,36 and projections 25,37 on the sample reservoir 20 and the base 30.

The lower end of the sample reservoir 20 is preferably formed with an annular sealing rim 26 which extends around the entire periphery of the sample reservoir 20. When the grooves 24, 36 and the projections 25, 37 of the sample reservoir 20 and the base 30 are engaged with each other, the sealing rim 26 is pressed downwards into sealing contact with the upper surface of the filter element 45 disposed atop the filter support surface 31 of the base 30. The compressive force between the sealing rim 26 and the filter element 45 is maintained by the engagement between the grooves 24, 36 and the projections 25, 37 of the sample reservoir 20 and the base 30. In the illustrated embodiment, the sealing rim 26 is positioned on the sample reservoir 20 such that an annular air space is present between the outer periphery of the sealing rim 26 and the inner periphery of the base 30 around the entire circumference of the sealing rim 26. It is thought that the air space may improve the integrity of the seal between the sample reservoir 20 and the base 30 by forming an air lock which prevents creeping of fluid by capillary action between the two members. However, the air space is not essential, and the sealing rim 26 may closely contact the inner periphery of the base 30.

A filter element 45 preferably comprises at least one filter medium compatible with the fluid being filtered and capable of removing microorganisms of interest from the fluid. The filter medium may be of any desired type, such as a microporous membrane or fibrous element of various materials, or filter paper, for example. For some applications, the filter medium is a thermally resistant material. A wide variety of filter media for microbiological studies are commercially available, and any such filter media can be employed with the present invention as the filter element 45. The filter medium may capture microorganisms in any desired manner, e.g., according to size, by adsorption, and/or affinity binding. Filter media for use in microbiological studies are frequently flat membrane discs, but the filter element 45 need not have any particular shape. For example, instead of being flat, the membrane may include pleats to increase its surface area.

The filter element 45 is disposed in a fluid flow path between the sample reservoir 20 and the fluid port 38 so that the fluid to be filtered passes through the filter element 45. The filter element may be self-supporting or alternatively as in the illustrated embodiment, the flat filter element 45 is supported by a filter support surface 31. The filter element 45 may be removably supported by the filter support surface 31 or may alternatively be permanently affixed to the filter support surface 31, for example by using an adhesive, a solvent, radio frequency sealing, ultrasonic sealing and/or heat sealing. Although in the illustrated embodiment, the base includes the filter support surface 31, alternatively the sample reservoir 20 may include a filter support surface. The filter element 45 may directly contact the filter support surface 31 or it may preferably rest upon an intermediate support member, which is more porous than the filter element 45 and which provides mechanical support to the filter element 45, such as a layer of mesh, paper or fabric. Alternatively, the filter element can comprise a filter medium laminated to a support. If the filter element 45 is to be left on the base 30 during incubation, it may be convenient if an absorbent pad 46 for use in holding a nutrient solution during incubation is placed beneath the filter element 45 prior to filtration rather than afterwards to reduce the amount of handling of the filter element 45 after filtration. Furthermore, the absorbent pad 46 may provide support for the filter element during filtration. It may also be desirable to place a prefilter, a protective sheet or other member atop the filter element 45.

It may be advantageous to place a resilient, compressible member between the lower surface of the filter element 45 and the filter support surface 31 in the region beneath where the sealing rim 26 contacts the filter element 45. Such a member can compensate for variations in the axial length of the sealing rim 26 and the filter support surface 31 to maintain the sealing rim 26 in intimate, sealing contact with the filter element 45, thereby enabling the manufacturing tolerances of the sample reservoir 20 and the base 30 to be less precise. The resilient member may be either permeable or impermeable to the fluid being filtered. For example, it may comprise an impermeable gasket disposed beneath the filter element 45. It is also possible to place a resilient sealing member, such as a gasket, between the top surface of the filter element 45 and the sealing rim 26 so that the sealing rim 26 does not directly contact the filter element 45 but is pressed into sealing contact with the sealing member, which in turn is pressed into sealing contact with the filter element 45. Such a sealing member may be separate from or joined to the filter element 45.

In the illustrated embodiment, the wall 34 surrounding the filter support surface 31 preferably has a height such that when an absorbent pad 46 and a filter element 45 are mounted on the filter support surface 31, the absorbent pad 46 will be surrounded by the wall 34 and disposed at least partially below the upper end of the wall 34, while the filter element 45 disposed atop the absorbent pad 46 will be positioned at or above the upper end of the wall 34 and will be surrounded by the radial projections 35. For example, the wall 34 may have a height substantially the same as the thickness of the absorbent pad 46. With the absorbent pad 46 located partially or entirely below the upper end of the wall 34, when a user of the filtration assembly 10 wishes to transfer the filter element 45 from atop the absorbent pad 46 to a different location, it is easy for the user to pick up the filter element 45 using forceps without picking up the absorbent pad 46 as well. The spaces between the radial projections 35 provide easy access to the filter element 45 and facilitate its removal from the base 30.

From the standpoint of ease of manufacture, it is preferable if the axial length of the sealing rim 26 of the sample reservoir 20 and the axial height of the radial projections 35 on the base 30 are such that when the sample reservoir 20 sealingly engages the base 30 and the sealing rim 26 of the sample reservoir 20 is pressed into sealing contact with the filter element 45 as shown in Figure 4, there is an axial gap between the top surface of the radial projections 35 and the bottom surface of the sample reservoir 20. If such a gap is present, the radial projections 35 and the sealing rim 26 do not need to be manufactured to as precise tolerances as when the upper surfaces of the radial projections 35 contact the bottom surface of the sample reservoir 20.

The filtration assembly 10 can be made from a wide variety of materials, including those conventionally used for funnels, reservoirs, petri dishes, and other laboratory equipment, such as metals, plastics, and glass, depending upon factors such as the desired strength, flexibility, heat resistance, and corrosion resistance and upon whether the filtration assembly 10 is intended to be reusable or discarded at the completion of use. Different portions of the filtration assembly 10 may be formed of different materials. For economy of manufacture, plastics which can be shaped by molding are particularly suitable for the filtration assembly 10. Some examples of suitable plastics are polypropylene, nylon, and polyacrylate. In some instances, it is convenient if portions of the assembly 10, such as the sample reservoir 20 are translucent or transparent to permit substances within the assembly 10 to be readily observed.

Typically, the filtration assemblies are shipped in a sealed container, such as a bag, while maintaining sterility. The filtration assembly can be sterilized in accordance with a variety of sterilization protocols known in the art.

A variety of fluids can be filtered in accordance with embodiments of the invention, e.g., fluids in the biopharmaceutical, microelectronics, and beverage industries. Embodiments of the invention are particularly useful where it is desirable to monitor the contamination of the fluid e.g., to ensure that the fluid is sterile. Embodiments of the invention are suitable for use in a variety of systems, including "hot loop" systems, e.g., wherein the fluid to be filtered is a heated fluid, e.g., heated to a temperature of about 80°C.

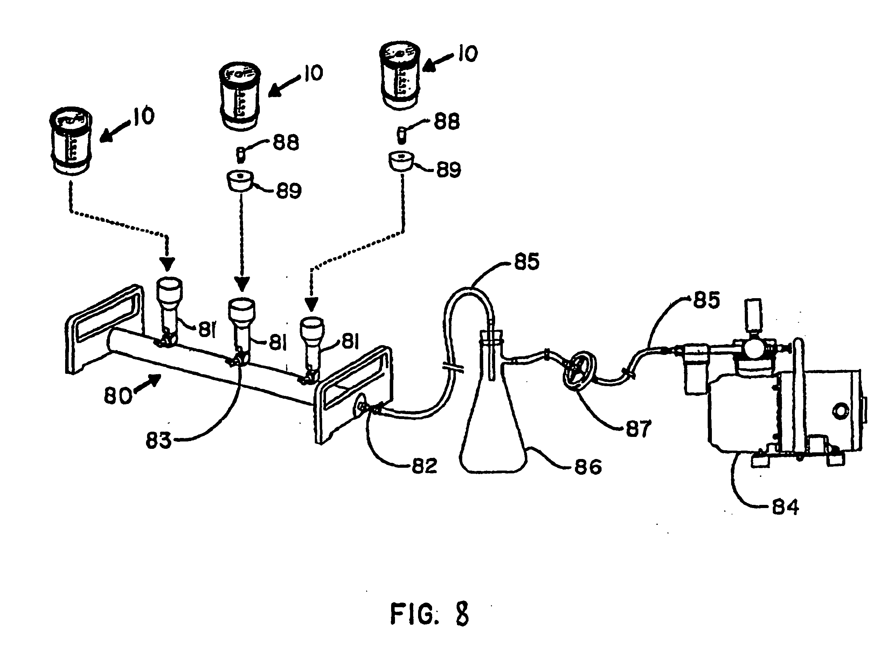

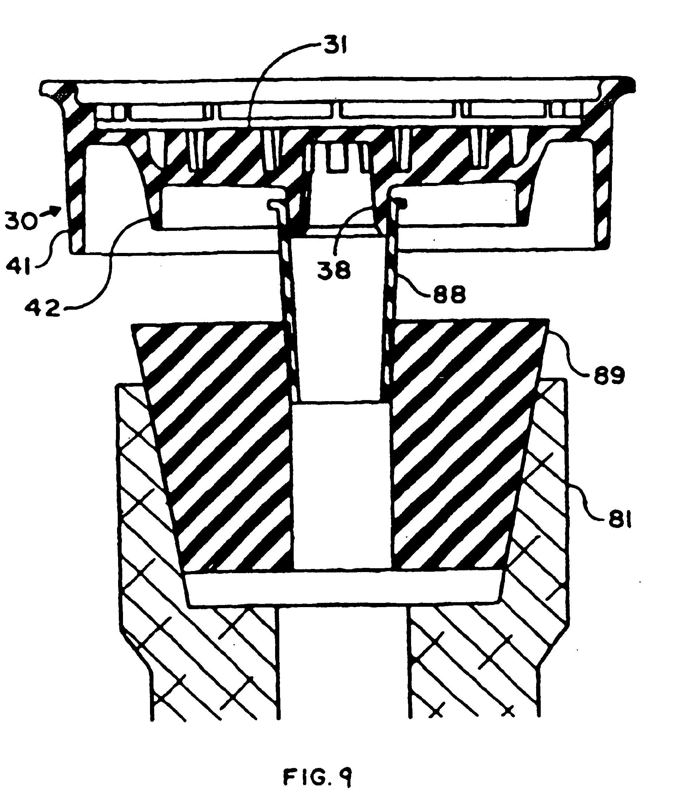

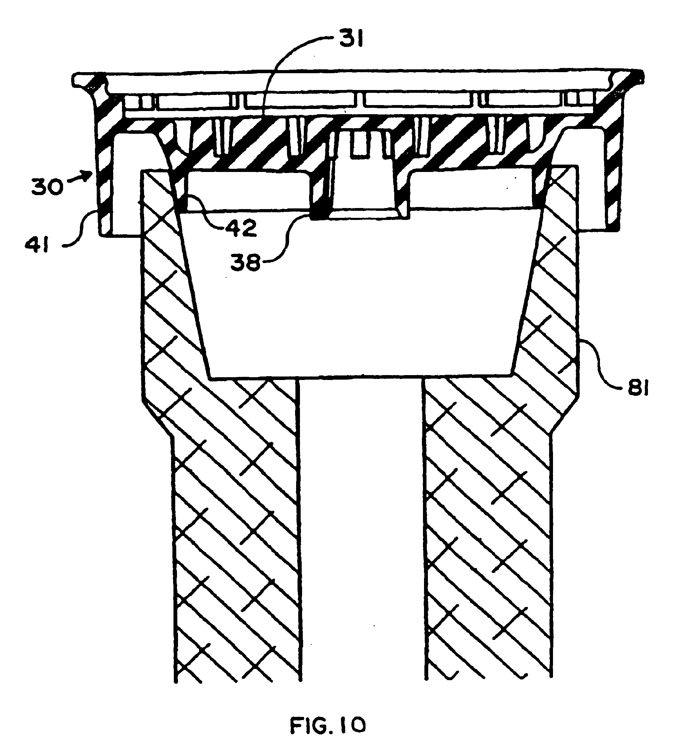

Filtration of a fluid sample in the sample reservoir 20 can be performed by a variety of conventional methods, including gravity filtration and vacuum filtration. In vacuum filtration, the filtration assembly 10 is mounted on a vacuum manifold, a filtration flask or other device through which suction can be applied to the fluid port 38 to suck fluid in the sample reservoir 20 through the filter element 45 and out of the fluid port 38, while drawing air into the sample reservoir 20 through the vent 70 and the liquophobic element 72. Figure 8 is a schematic view of a vacuum filtration arrangement with which a filtration assembly 10 according to the present invention can be employed. The illustrated arrangement includes a vacuum filtration manifold 80 having a plurality of inlet tubes 81, each of which can support a filtration assembly 10. Any one of the inlet tubes 81 can be fluidly connected through the interior of the manifold 80 to a vacuum port 82 of the manifold 80 by a stopcock. Suction can be applied to the vacuum port 82 by a vacuum pump 84 connected to it by a hose 85. Depending on the structure of the pump 84, a vacuum filtration flask 86 and a filter 87 for removing aerosols from the air may be installed between the manifold 80 and the pump 84 to prevent the fluid being filtered from being sucked into the pump 84. In order to perform filtration with this arrangement, a filtration assembly 10 containing a filter element 45 and possibly an absorbent pad 46 disposed on the filter support surface 31 of the base 30 is mounted on one of the inlet tubes 81 with the fluid port 38 of the base 30 fluidly communicating with the inlet tube 81. The fluid port 38 may be connected to one of the inlet tubes 81 in a variety of manners. One way, schematically shown in Figure 9 is to insert the fluid port 38 into the upper end of a hollow adapter 88 and to insert the lower end of the adapter 88 into the bore of a hollow rubber stopper 89 sized to fit into the upper end of one of the inlet tubes 81. The adapter 88, which may be either a rigid or flexible member, is sized so as to form line or surface contact with the outer surface of the fluid port 38 when the fluid port 38 is inserted into the adapter 88 with a sufficiently tight fit between the fluid port 38 and the adapter 88 to obtain a desired suction in the fluid port 38 when the vacuum pump 84 is operated. Alternatively, as schematically shown in Figure 10, the base 30 of the filtration assembly 10 may also be shaped so as to directly engage with the inlet tube 81 of the manifold 80 without the need for an adapter 88 or a stopper 89.

In the embodiment shown in Figure 10, the base 30 includes an annular skirt 42 disposed between the fluid port 38 and the outer wall 41 and extending downwards from the lower surface of the base 30. The outer periphery of the skirt 42 is shaped so as to be in line contact or surface contact with the inner surface of the inlet tube 81 around its entire periphery when the skirt 42 is inserted into the inlet tube 81. The skirt 42 may but need not form a fluid-tight seal against the inlet tube 81. The skirt 42 preferably engages the inlet tube 81 sufficiently tightly that the vacuum pump 84 can generate sufficient suction in the inlet tube 81 to suck fluid contained in the sample reservoir 20 through the filter element 45. It may be easier to obtain a desired fit between the skirt 42 and the inlet tube 81 if the skirt 42 is somewhat flexible. The skirt 42 may also be shaped to directly contact filtration equipment other than an inlet tube of a vacuum filtration manifold, such as the mouth of a filtration flask. Preferably, the fluid sample to be filtered is originally collected in the sample reservoir 20, alternatively, the fluid may be placed in the sample reservoir after collection, and either before or after the assembly 10 is mounted on the inlet tube 81. With the filtration assembly 10 mounted on one of the inlet tubes 81 and cover 50 fluid-tightly engaged with the reservoir 20, the vacuum pump 84 is operated to suck the fluid sample through the filter element 45 and into the filtration flask. During operation of the pump 84, the cover need not be removed since the vent allows air to pass into the sample reservoir, and the vent has a pore structure that allows air, but not contaminants (e.g., microorganisms such as bacteria) from the environment external to the sample reservoir to pass into the reservoir. When the fluid sample has been sucked out of the sample reservoir 20 and through the filter element 45, the pump 84 is turned off. At this time, the filtration assembly 10 may be removed from or left mounted on the vacuum manifold 80.

The filter element can be removed from the filter assembly and transferred to a petri dish wherein the captured microorganisms, if present, can be cultured. In those embodiments wherein the base 30 and cover 50 or base 30 and additional cover are to be used as a petri dish, after the completion of filtration, the sample reservoir 20 is detached by hand from the base 30 by releasing the snap fit between them, and the filter element 45 (that can be removable, or permanently affixed to the filter support surface 31) is left atop the base 30 where a suitable nutrient solution is applied to the absorbent pad 46 located beneath the filter element 45, the absorbent pad 46 typically having been placed beneath the filter element 45 prior to filtration.

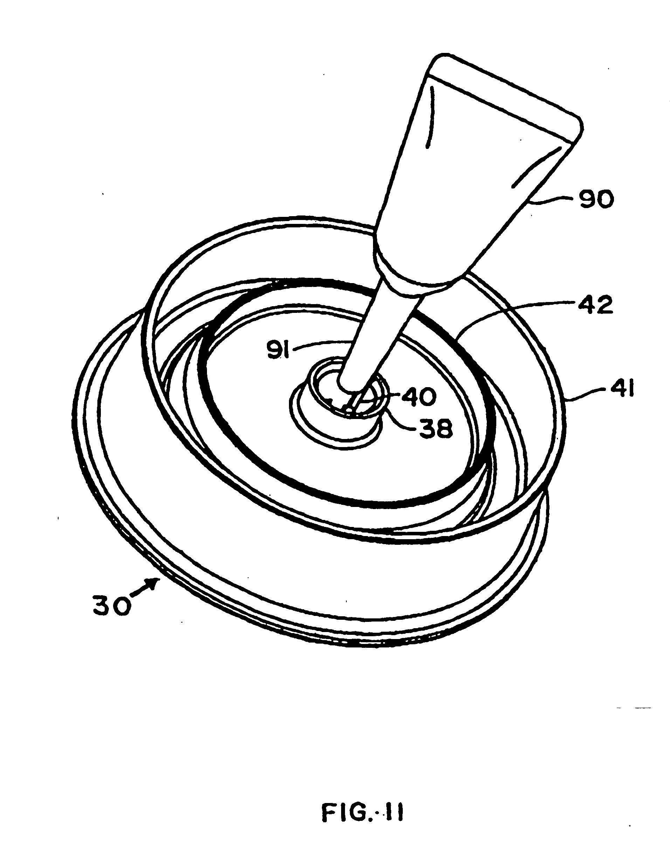

The nutrient solution can be applied to the absorbent pad 46, either from above through the filter element 45 or from below via the fluid port 38. A method of introducing the solution through the fluid port 38 is shown in Figure 11. The nutrient solution is usually contained in an ampoule 90 having a tapered snout 91 which can be inserted into the fluid port 38 and from which the nutrient solution can be dispensed. Since the fit between the outer surface of the snout 91 of the ampoule 90 and the inner surface of the fluid port 38 may be fairly tight, one or more air vents 40 may be formed in the fluid port 38 to enable air to escape from the fluid port 38 when the outer surface of the snout 91 of the ampoule 90 is pressed tightly against the inner surface of the fluid port 38 to prevent the formation of an air lock which could impede the introduction of the nutrient solution into the fluid port 38. In the illustrated embodiment, the fluid port 38 has three air vents 40, each comprising an elongated groove formed in the inner periphery of the fluid port 38 between the openings 39 in the fluid port 38 and its outer end. However, the fluid port may alternatively include more than three or fewer than three air vents, and they need not comprise an elongated groove. When the nutrient solution is being applied to the absorbent pad 46 through the fluid port 38, the sample reservoir 20, the cover 50, or the additional cover may be mounted on the base 30 to prevent the filter element 45 and absorbent pad 46 from falling off. Once the nutrient solution has been applied to the absorbent pad 46 through the fluid port 38, the petri dish comprising the base 30 and the cover 50 or the base 30 and additional cover member are ready to be incubated. If desired, a closure, such as a cap or a plug, may be mounted on the lower end of the fluid port 38 to prevent fluid from leaking out of it during incubation.

In accordance with an embodiment of the invention, a test kit is provided, comprising the filtration assembly, and one or more of a nutrient solution, a growth medium, and a reagent (e.g., for detecting the presence of the microorganism(s)). Preferably, the test kit includes a sterile filtration assembly sealed in one container, while the nutrient solution, growth medium and/or reagent(s) are sealed in another container.

All of the references cited herein, including publications, patents, and patent applications, are hereby incorporated in their entireties by reference.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Of course, variations of those preferred embodiments will become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A filtration assembly comprising:
a sample reservoir for holding a fluid sample to be filtered;
a cover member detachably covering the sample reservoir with a fluid-tight fit; at least one vent in communication with the sample reservoir, wherein the vent includes a liquophobic element that allows air into the sample reservoir but prevents the passage of microorganisms into the sample reservoir;
a fluid port in fluid communication with the sample reservoir;
a filter element disposed in a flow path between the sample reservoir and the fluid port.

2. The filtration assembly according to claim 1, wherein the cover member includes the vent.

3. The filtration assembly according to claim 1, wherein the sample reservoir includes the vent.

4. The filtration assembly according to claim 1, including a base detachably mounted on the sample reservoir, the base including the fluid port, and a filter support surface for supporting the filter element.

5. The filtration assembly according to claim 4, wherein the cover member includes said vent communicating with the sample reservoir.

6. The filtration assembly according to claim 5, further comprising a filter element supported by the filter surface and disposed in a fluid flow path between the sample reservoir and the fluid port.

7. The filtration assembly according to any of claims 1-6, wherein the filter element is removably disposed in a fluid flow path between the sample reservoir and the fluid port.

8. The filtration assembly according to any of claims 1-7, wherein the liquophobic element comprises a microporous membrane.

9. The filtration assembly according to any of claims 1 to 7, wherein the filter element comprises a microporous membrane.

10. A method of filtering a fluid comprising:
collecting a fluid to be filtered in a sample reservoir of a filtration assembly according to any one of claims 1 to 9;
connecting the detachable cover member to the sample reservoir;
drawing the fluid to be filtered through the filter element disposed adjacent the sample reservoir and passing air into the reservoir through the vent; and
removing the fluid which has passed through the filter element from the fluid port.

11. The method according to claim 10, wherein passing air into the reservoir through the vent comprises passing air into the reservoir through a vent disposed in the cover member.

12. The method according to claim 10 or 11, further comprising applying suction to draw the fluid through the filter element and remove the fluid through the fluid port.

13. The method according to any of claims 10 to 12, further comprising incubating microorganisms retained on the filter element after removing the fluid which has passed through the filter element.

14. The method of filtering a fluid according to claim 10, wherein said fluid is a microorganism-containing fluid wherein said filter element comprises a microorganism-capturing filter medium;
and wherein said fluid port communicates with the sample reservoir;
said method comprising a further step of incubating the microorganisms captured by the filter medium.

15. Use of a filtration assembly according to claim 5, said assembly comprising a base with a skirt, said use including
placing said filtration assembly on a vacuum manifold with said skirt of the base contacting an inlet tube of the manifold around a periphery of the skirt,
applying suction to the interior of the inlet tube to draw a fluid to be filtered through a filter element within the filtration assembly and into the manifold and to draw air through the vent into the filtration assembly.

## Patentansprüche

1. Filtrationsanordnung, umfassend:
ein Probenreservoir zur Aufnahme einer Fluid-Probe, die zu filtrieren ist;
ein Deckelelement, welches lösbar das Probenreservoir fluiddicht abdeckt; mindestens einen Durchlass, der in Verbindung mit dem Probenreservoir steht, wobei der Durchlass ein liquophobes Element umfasst, welches Luft in das Probenreservoir hineinlässt, jedoch verhindert, dass Mikroorganismen in das Probenreservoir gelangen;
eine Fluidöffnung in Fluidverbindung mit dem Probenreservoir;
ein Filterelement, welches in dem Fließweg zwischen dem Probenreservoir und der Fluidöffnung angeordnet ist.

2. Filtrationsanordnung nach Anspruch 1, worin das Deckelelement den Durchlass umfasst.

3. Filtrationsanordnung nach Anspruch 1, worin das Probenreservoir den Durchlass umfasst.

4. Filtrationsanordnung nach Anspruch 1, umfassend eine Basis, welche lösbar an dem Probenreservoir gehalten ist, wobei die Basis die Fluidöffnung und eine filterstützende Oberfläche, um das Filterlement zu stützen, umfasst.

5. Filtrationsanordnung nach Anspruch 4, worin das Deckelelement den Durchlass, der mit dem Probenreservoir in Verbindung steht, umfasst.

6. Filtrationsanordnung nach Anspruch 5, welche ferner ein Filterelement umfasst, welches durch die Filteroberfläche getragen wird und welches in dem Fluidfließweg zwischen dem Probenreservoir und der Fluidöffnung angeordnet ist.

7. Filtrationsanordnung nach einem der Ansprüche 1 bis 6, worin das Filterelement entfernbar in dem Fluidfließweg zwischen dem Probenreservoir und der Fluidöffnung angeordnet ist.

8. Filtrationsanordnung nach einem der Ansprüche 1 bis 7, worin das liquophobe Element eine mikroporöse Membran umfasst.

9. Filtrationsanordnung nach einem der Ansprüche 1 bis 7, worin das Filterelement eine mikroporöse Membran umfasst.

10. Verfahren zum Filtern eines Fluids, umfassend:
Sammeln eines Fluids, das zu filtrieren ist, in einem Probenreservoir einer Filtrationsanordnung gemäß einem der Ansprüche 1 bis 9;
Verbinden des abnehmbaren Deckelelements mit dem Probenreservoir;
Durchsaugen des zu filternden Fluids durch das Filterelement, welches benachbart zu dem Probenreservoir angeordnet ist, und Durchleiten von Luft in das Reservoir durch den Durchlass; und
Entfernen des Fluids, welches durch das Filterelement hindurchgeleitet wurde, durch die Fluidöffnung.

11. Verfahren nach Anspruch 10, worin das Durchleiten von Luft in das Reservoir das Durchleiten von Luft in das Reservoir durch einen Durchlass umfasst, welcher in dem Deckelelement angeordnet ist.

12. Verfahren nach Anspruch 10 oder 11, welches ferner das Anwenden von Unterdruck umfasst, um das Fluid durch das Filterelement hindurchzusaugen und das Fluid durch die Fluidöffnung zu entfernen.

13. Verfahren nach einem der Ansprüche 10 bis 12, ferner umfassend das Inkubieren von Mikroorganismen, die auf dem Filterelement zurückgehalten wurden, nachdem das Fluid, welches durch das Filterelement hindurchgetreten ist, entfernt wurde.

14. Verfahren zum Filtern eines Fluids nach Anspruch 10, worin das Fluid ein Mikroorganismen enthaltendes Fluid ist, worin das Filterelement ein Mikroorganismen rückhaltendes Filtermedium umfasst; und worin die Fluidöffnung in Verbindung mit dem Probenreservoir steht; wobei das Verfahren den weiteren Schritt des Inkubierens der Mikroorganismen, die von dem Filterelement zurückgehalten wurden, umfasst.

15. Verwendung einer Filtrationsanordnung nach Anspruch 5, wobei die Anordnung eine Basis mit einem Rand umfasst, wobei diese Verwendung umfasst
Platzieren der Filtrationsanordnung auf einer Vakuumvorrichtung, wobei der Rand der Basis mit einem Einlassrohr der Vorrichtung rings um einen Außenumfang des Randes in Kontakt steht;
Anwenden von Unterdruck auf das Innere des Einlassrohres, um zu filterndes ein Fluid durch ein Filterelement innerhalb der Filtrationsanordnung hindurch- und in die Vorrichtung hineinzusaugen und um Luft durch den Durchlass in die Filtrationsanordnung zu saugen.

## Revendications

1. Ensemble de filtration comprenant :
un réservoir d'échantillon destiné à contenir un échantillon fluide destiné à être filtré ;
un élément de couvercle couvrant de manière amovible le réservoir d'échantillon avec un ajustement étanche ; au moins un évent en communication avec le réservoir d'échantillon, dans lequel l'évent comprend un élément liquidophobe qui permet à l'air de passer dans le réservoir d'échantillon mais empêche le passage de micro-organismes dans le réservoir d'échantillon ;
un orifice de fluide en communication de fluide avec le réservoir d'échantillon ;
un élément de filtre placé dans une voie de passage entre le réservoir d'échantillon et l'orifice de fluide.

2. Ensemble de filtration selon la revendication 1, dans lequel l'élément de couvercle comprend l'évent.

3. Ensemble de filtration selon la revendication 1, dans lequel le réservoir d'échantillon comprend l'évent.

4. Ensemble de filtration selon la revendication 1, comprenant une base montée de manière amovible sur le réservoir d'échantillon, la base comprenant l'orifice de fluide, et une surface de support de filtre destinée à supporter l'élément de filtre.

5. Ensemble de filtration selon la revendication 4, dans lequel l'élément de couvercle comprend ledit évent communiquant avec le réservoir d'échantillon.

6. Ensemble de filtration selon la revendication 5, comprenant en outre un élément de filtre supporté par la surface de filtre et placé dans une voie de passage de fluide entre le réservoir d'échantillon et l'orifice de fluide.

7. Ensemble de filtration selon l'une quelconque des revendications 1 à 6, dans lequel l'élément de filtre est placé de manière amovible dans une voie de passage de fluide entre le réservoir d'échantillon et l'orifice de fluide.

8. Ensemble de filtration selon l'une quelconque des revendications 1 à 7, dans lequel l'élément liquidophobe comprend une membrane microporeuse.

9. Ensemble de filtration selon l'une quelconque des revendications 1 à 7, dans lequel l'élément de filtre comprend une membrane microporeuse.

10. Procédé de filtration d'un fluide comprenant :
la collecte d'un fluide destiné à être filtré dans un réservoir d'échantillon d'un ensemble de filtration selon l'une quelconque des revendications 1 à 9 ;
la connexion de l'élément de couvercle amovible au réservoir d'échantillon ;
l'attraction du fluide destiné à être filtré à travers l'élément de filtre placé à côté du réservoir d'échantillon et l'acheminement d'air dans le réservoir à travers l'évent ; et
le retrait du fluide qui est passé à travers l'élément de filtre par l'orifice de fluide.

11. Procédé selon la revendication 10, dans lequel l'acheminement d'air dans le réservoir à travers l'évent comprend l'acheminement d'air dans le réservoir à travers un évent placé dans l'élément de couvercle.

12. Procédé selon la revendication 10 ou 11, comprenant en outre l'application d'une aspiration pour attirer le fluide à travers l'élément de filtre et retirer le fluide à travers l'orifice de fluide.

13. Procédé selon l'une quelconque des revendications 10 à 12, comprenant en outre l'incubation de micro-organismes retenus sur l'élément de filtre après ie retrait du fluide qui est passé à travers l'élément de filtre.

14. Procédé de filtration d'un fluide selon la revendication 10, dans lequel ledit fluide est un fluide contenant des micro-organismes dans lequel ledit élément de filtre comprend un milieu filtrant de capture de micro-organismes ;
et dans lequel ledit orifice de fluide communique avec le réservoir d'échantillon ;
ledit procédé comprenant une étape supplémentaire d'incubation des micro-organismes capturés par le milieu filtrant.

15. Utilisation d'un ensemble de filtration selon la revendication 5, ledit ensemble comprenant une base avec une jupe, ladite utilisation comprenant
le placement dudit ensemble de filtration sur un collecteur pneumatique avec ladite jupe de la base entrant en contact avec un tuyau d'arrivée du collecteur autour d'une périphérie de la jupe,
l'application d'une aspiration à l'intérieur du tuyau d'arrivée pour attirer un fluide destiné à être filtré à travers un élément de filtre à l'intérieur de l'ensemble de filtration et dans le collecteur et pour attirer de l'air à travers l'évent dans l'ensemble de filtration.
